# FASCICULE DE BREVET EUROPEEN

(11) **EP 3 119 298 B1**
(45) Date de publication et mention de la délivrance du brevet: **07.02.2018**
(21) Numéro de dépôt: 15714571.5
(22) Date de dépôt: 04.03.2015
(51) Int. Cl.: A61B 17/70

(54) **ENSEMBLE CHIRURGICAL, VIS D'ANCRAGE OSSEUX ET DISPOSITIF D'EXTENSION D'UNE TELLE VIS FAISANT PARTIES DUDIT ENSEMBLE CHIRURGICAL**
CHIRURGISCHE ANORDNUNG UND KNOCHENANKERSCHRAUBE UND VORRICHTUNG ZUR ERWEITERUNG SOLCH EINER SCHRAUBE, DIE ZUR BESAGTEN CHIRURGISCHEN ANORDNUNG GEHÖRT
SURGICAL ASSEMBLY, AND BONE ANCHOR SCREW AND DEVICE FOR EXTENDING ONE SUCH SCREW THAT BELONG TO SAID SURGICAL ASSEMBLY

(30) Priorité: 20.03.2014 FR 1452299
(43) Date de publication de la demande: 25.01.2017
(73) Titulaire: Spineway, 69130 Ecully (FR)
(72) Inventeur: LE ROUX, Stéphane, F-69002 Lyon (FR); LAURITO, Philippe, F-83143 Le Val (FR); BAZILLE, Julien, F-69009 Lyon (FR)
(74) Mandataire: Cabinet Laurent & Charras
(86) Numéro de dépôt international: PCT/FR2015/050535
(87) Numéro de publication internationale: WO 2015/140440

(56) Documents cités:
- EP-A1- 2 692 304
- US-A1- 2005 131 408
- US-A1- 2013 245 702
- US-A1- 2014 052 187

## Description

### DOMAINE TECHNIQUE

La présente invention concerne le secteur technique des instruments chirurgicaux.
L'invention trouve une application avantageuse pour le traitement des instabilités chroniques, des fractures ou des déformations du rachis particulièrement pour un abord postérieur mini-invasif.
Cependant, l'invention peut être transposée dans toutes les applications dans lesquelles une vis d'ancrage osseux est implantée par un chirurgien.

### ETAT ANTERIEUR DE LA TECHNIQUE

Dans le domaine de l'ostéosynthèse rachidienne, il est parfaitement connu d'utiliser des implants rachidiens mettant en oeuvre au moins deux vis d'ancrage osseux, notamment des vis pédiculaires, destinées à être vissées dans la vertèbre, et un système de liaison (plaque ou tige) unissant les deux vis pédiculaires entre elles afin de réaliser une distraction ou compression, et redéfinir la courbure de la colonne vertébrale. La tête d'une vis d'ancrage osseux se présente généralement sous la forme d'une douille apte à recevoir transversalement ladite tige de liaison et longitudinalement un organe de verrouillage pour verrouiller la tige de liaison dans la tête de la vis.

Dans le cadre d'une implantation par un abord mini-invasif, l'implantation et la manipulation de ces vis pédiculaires sont difficiles pour le chirurgien car l'accès est relativement étroit. Il est d'usage d'utiliser des dispositifs d'extension d'une vis pédiculaire pour aider le chirurgien, d'une part, à l'implantation et à la manipulation des vis pédiculaires, et, d'autre part, à la mise en place des tiges de liaison desdites vis pédiculaires.
Le dispositif d'extension et la vis pédiculaire peuvent être adaptés l'un à l'autre de sorte à former un ensemble chirurgical pour le traitement d'instabilités, de fractures ou de déformations d'une colonne vertébrale.

Un dispositif d'extension connu se présente sous la forme d'un élément tubulaire dont l'une des extrémités est apte à être fixée temporairement à la tête de la vis pédiculaire. L'élément tubulaire est tel qu'il laisse libre l'accès à la tête de vis, et autorise l'insertion d'un organe de verrouillage d'une tige de liaison, et d'un outil pour le verrouillage dudit organe de verrouillage.

L'extrémité de l'élément tubulaire peut présenter à cet effet une forme de pince apte à enserrer la paroi externe de la tête de vis pédiculaire.

Les pinces peuvent comprendre sur leurs surfaces internes en regard de la tête de vis, des agencements aptes à coopérer avec des agencements complémentaires ménagés sur la tête de vis afin de verrouiller temporairement la coopération entre ladite tête de vis et le dispositif d'extension.

Cependant, de tels agencements ne sont souvent pas suffisants pour maintenir d'une manière optimale la tête de vis en engagement à l'extrémité du dispositif d'extension.

Des dispositifs de l'état de la technique sont divulgués dans les documents US 2013/245702, US 2005/131408, EP 2 692 304 et US 2014/052187.

### EXPOSE DE L'INVENTION

La présente invention est définie par la revendication indépendante 1 tandis que des modes de réalisations préférés sont exposés dans les revendications dépendantes.
L'un des buts de l'invention est donc de remédier au moins aux inconvénients précités en proposant un ensemble chirurgical, comprenant une vis d'ancrage osseux et un dispositif d'extension qui permettent de conserver l'accès à la douille de la vis d'ancrage osseux pendant l'opération chirurgicale, tout en assurant un maintien temporaire optimal dudit dispositif d'extension sur ladite tête de vis.

Un autre objectif de l'invention est de fournir un dispositif d'extension qui permette de faciliter, la mise en place des tiges de liaison de vis d'ancrage osseux, ainsi que la mise en place des organes de verrouillage desdites tiges de liaison dans la douille de la vis d'ancrage osseux.

A cet effet, il a donc été mis au point un ensemble chirurgical tel que par exemple pour le traitement d'instabilités, de fractures ou de déformations de la colonne vertébrale, ledit ensemble comprenant :
- une vis d'ancrage osseux présentant une partie d'ancrage dans la colonne vertébrale, et une tête sous forme d'une douille, et ;
- un dispositif d'extension destiné à venir coiffer la douille de la vis d'ancrage osseux pour en réaliser un prolongement longitudinal.

Selon l'invention, le dispositif d'extension comprend un premier élément tubulaire, dit tube interne, de diamètre interne supérieur ou égal au diamètre interne de la douille de la vis d'ancrage osseux à coiffer. Ce tube interne se prolonge longitudinalement par deux branches diamétralement opposées. Lesdites branches comprennent une élasticité les autorisant à être écartées ou rapprochées l'une de l'autre. Les extrémités libres des deux branches forment une pince apte à pincer la surface externe de la douille pour se verrouiller sur celle-ci.

Toujours selon l'invention, le dispositif d'extension comprend un deuxième élément tubulaire, dit tube externe, monté autour du tube interne, et de diamètre interne ajusté au diamètre externe dudit tube interne de manière à pouvoir coulisser le long dudit tube interne, entre une position dite de non verrouillage et une position dite de verrouillage de la vis d'ancrage osseux. Le tube externe se prolonge longitudinalement par deux branches diamétralement opposées et superposées aux branches du tube interne. Les extrémités libres des deux branches du tube externe sont en regard des extrémités libres des deux branches du tube interne, et comprennent chacune des moyens de retenue aptes à coopérer, lorsque le dispositif coiffe la douille de la vis d'ancrage osseux, et lorsque ledit tube externe est coulissé dans sa position de verrouillage, avec des moyens de retenue complémentaires agencés sur la douille pour empêcher lesdites branches du tube externe de s'écarter l'une de l'autre, empêchant ainsi les branches du tube interne de s'écarter l'une de l'autre de sorte à permettre le maintien du verrouillage du dispositif d'extension sur la douille de la vis d'ancrage osseux.

De cette manière, le dispositif d'extension peut coiffer la douille d'une vis d'ancrage osseux de manière à en réaliser un prolongement longitudinal pour faciliter la manipulation et l'implantation de la vis d'ancrage osseux. Le diamètre interne du tube interne est supérieur ou égal au diamètre interne de la douille, de sorte que le dispositif d'extension permette de conserver l'accès à la douille de la vis d'ancrage osseux par l'intérieur dudit tube interne. Le verrouillage du dispositif d'extension sur la douille est optimal. Les tubes interne et externe se prolongent par des branches diamétralement opposées de sorte à libérer un espace entre lesdites branches pour autoriser la mise en place d'une tige de liaison transversale pouvant être coulissée jusqu'à la douille de la vis d'ancrage osseux pour sa mise en place.

Selon une forme de réalisation particulière, les moyens de retenue complémentaires sont des surfaces de contact ménagées aux extrémités des branches du tube externe et sur la douille. Lesdites surfaces de contact sont destinées à venir en appui les unes contre les autres lorsque le dispositif coiffe une vis d'ancrage osseux, et lorsque le tube externe est coulissé dans sa position de verrouillage pour empêcher les branches du tube externe de s'écarter l'une de l'autre.

Avantageusement, des moyens de fixation complémentaires aptes à verrouiller le déplacement longitudinal desdites branches du tube interne par rapport à la surface externe de la douille sont agencés, d'une part, aux extrémités libres des branches du tube interne, et sur leur surface interne destinée à être en contact avec la surface externe de la douille, et, d'autre part, sur ladite surface externe de la douille.

De cette manière, le verrouillage du dispositif d'extension sur la douille est d'autant plus efficace. En effet, les moyens de fixation complémentaires sont aptes à verrouiller le déplacement longitudinal des branches du tube interne, de sorte qu'il devient alors nécessaire d'écarter lesdites branches l'une de l'autre pour déverrouiller le dispositif. Or, en position de verrouillage, le tube externe empêche lesdites branches du tube interne de s'écarter l'une de l'autre. Le verrouillage est optimal

Selon une forme de réalisation particulière, les moyens de fixation complémentaires sont du type ergots et logements complémentaires aptes à coopérer les uns avec les autres.

L'invention concerne également et séparément, un dispositif d'extension, et une vis d'ancrage osseux, comprenant les caractéristiques précitées et faisant parties d'un ensemble chirurgical tel que précité.

Avantageusement, dans le dispositif d'extension selon l'invention, au moins l'une des branches du tube interne présente à son extrémité libre un épaulement transversal apte à réaliser une butée contre la douille d'une vis d'ancrage osseux lorsque ledit dispositif vient coiffer ladite douille.

Selon une autre caractéristique, le dispositif d'extension comprend un organe de verrouillage apte à maintenir le tube externe en position de verrouillage de la vis d'ancrage osseux.

D'une manière avantageuse, le tube interne et le tube externe comprennent en outre des moyens de guidage complémentaires aptes à empêcher la rotation du tube externe par rapport au tube interne.

Par ailleurs, les branches du tube interne comprennent chacune sur leur surface interne un bossage ou épaulement. Lesdits bossages sont diamétralement opposés, et en regard l'un de l'autre de sorte que lorsqu'un outil adapté est inséré à l'intérieur du tube interne, celui-ci vient buter contre lesdits bossages pour forcer les branches du tube interne à s'écarter l'une de l'autre.

De préférence, le dispositif d'extension selon l'invention inclut également l'outil précité sous la forme d'une tige apte à être introduite dans le tube interne dudit dispositif pour venir buter contre les bossages ou épaulements et forcer les branches du tube interne à s'écarter l'une de l'autre.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :
- la figure 1 est une représentation schématique en perspective d'un ensemble chirurgical pour le traitement d'instabilités, de fractures ou de déformations d'une colonne vertébrale selon l'invention, avant que le dispositif d'extension ne coiffe la vis d'ancrage osseux, notamment la vis pédiculaire ;
- la figure 2 est une représentation schématique en perspective d'un outil d'aide à la mise en place d'un dispositif d'extension selon l'invention ;
- la figure 3 est une représentation schématique de face, illustrant en détail l'extrémité du dispositif d'extension avant qu'il ne coiffe la douille d'une vis pédiculaire, avec la présence de l'outil d'aide à la mise en place ;
- la figure 4 est une représentation schématique similaire à celle de la figure 3, illustrant en coupe longitudinale et de face, la mise en place du dispositif d'extension sur la douille d'une vis pédiculaire ;
- la figure 5 est une représentation schématique de face, illustrant le dispositif d'extension mis en place sur la douille d'une vis pédiculaire, avant le verrouillage des branches du tube interne sur la surface externe de ladite douille ;
- la figure 6 est une représentation schématique de face similaire à celle de la figure 5, l'outil d'aide à la mise en place étant retiré et les branches étant verrouillées sur la surface externe de la douille ;
- la figure 7 est une représentation schématique similaire à celle de la figure 6, l'ensemble chirurgical étant représenté en coupe longitudinale et de face ;
- la figure 8 est une représentation schématique en perspective, illustrant l'ensemble chirurgical selon l'invention, le dispositif d'extension coiffant la douille d'une vis pédiculaire ;
- la figure 9 est une représentation schématique en perspective similaire à celle de la figure 8, illustrant en détail la coopération entre le dispositif d'extension et la vis pédiculaire ;
- la figure 10 est une représentation schématique en perspective similaire à celle de la figure 8, le tube externe du dispositif d'extension étant coulissé dans sa position de verrouillage de la vis pédiculaire ;
- la figure 11 est une représentation schématique en perspective similaire à celle de la figure 10, illustrant en détail la coopération entre le tube externe dans sa position de verrouillage et la vis pédiculaire ;
- la figure 12 est une représentation schématique similaire à celle de la figure 11, illustrant l'ensemble vue de face ;
- la figure 13 est une représentation schématique en perspective similaire à celle de la figure 10, illustrant le verrouillage de la position de verrouillage du tube externe du dispositif d'extension ;
- la figure 14 est une représentation schématique illustrant en perspective l'extrémité du dispositif d'extension sur le point de recevoir un organe de verrouillage sous la forme d'une bague ouverte ;
- la figure 15 est une représentation schématique similaire à celle de la figure 14, la bague étant encliquetée à l'extrémité du dispositif d'extension ;
- la figure 16 est une représentation schématique illustrant le dispositif selon l'invention comprenant une ouverture longitudinale ménagée tout le long dudit dispositif.

### EXPOSE DETAILLE DE L'INVENTION

L'invention concerne un ensemble chirurgical (1). A titre d'exemple, il est décrit ci-dessous un ensemble chirurgical (1) pour le traitement d'instabilités, de fractures ou de déformations de la colonne vertébrale. Cependant il est évident que ledit ensemble chirurgical peut être transposé dans toutes les applications dans lesquelles une vis d'ancrage osseux est implantée par un chirurgien. Dans l'application détaillée ci-après, la vis d'ancrage osseux est une vis pédiculaire.

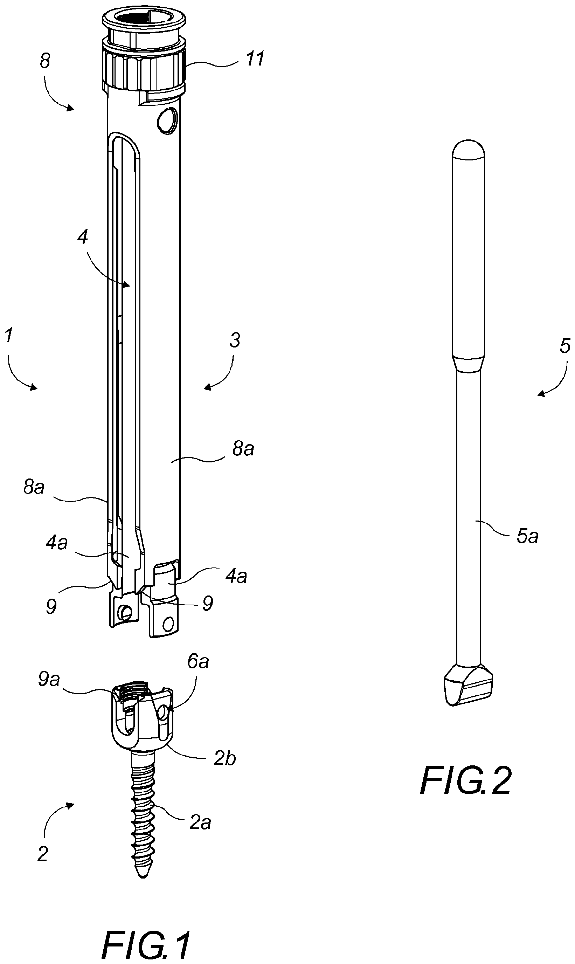

En référence à la figure 1, l'ensemble (1) comprend séparément, une vis pédiculaire (2) et dispositif d'extension (3) de la vis pédiculaire (2), adaptés pour coopérer temporairement l'un avec l'autre le temps d'une opération chirurgicale de traitement d'instabilités, de fractures ou de déformations de la colonne vertébrale.

La vis pédiculaire (2) comprend une partie d'ancrage (2a) dans la colonne vertébrale, et une tête sous forme d'une douille (2b). La douille (2b) est apte à recevoir transversalement une tige de liaison, et un organe de verrouillage de la tige de liaison (non représentés), faisant parties d'un implant rachidien bien connu de l'homme du métier. La douille (2b) de la vis pédiculaire (2) est apte à recevoir le dispositif d'extension (3) selon l'invention.

Le dispositif d'extension (3) comprend un premier élément tubulaire, dit tube interne (4). Afin de libérer l'accès à la douille (2b) depuis l'intérieur dudit tube interne (4), le tube interne (4) comprend un diamètre interne supérieur ou égal au diamètre interne de la douille (2b) à coiffer. Le tube interne (4) autorise alors l'insertion, d'un outil (non représenté) pour manipuler la tête de la vis pédiculaire (2), d'un outil (5) pour aider à la mise en place ou le retrait du dispositif d'extension (3) sur la douille (2b), ou bien d'un organe de verrouillage (non représenté) destiné à coopérer avec la tête de vis pour verrouiller une tige de liaison de deux vis pédiculaires (2).

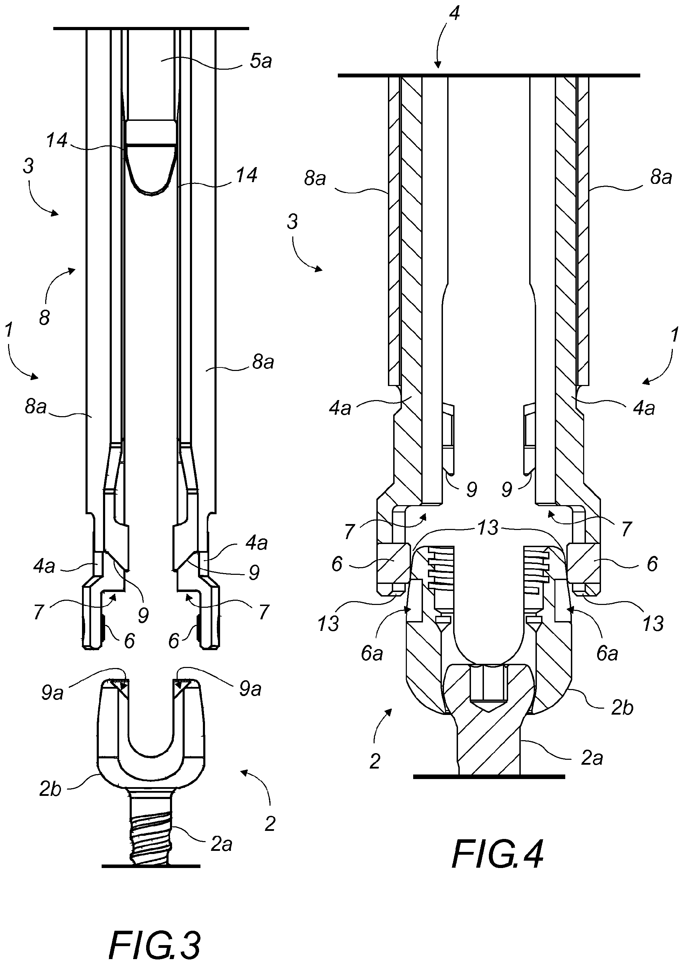

En référence aux figures 3 et 4, le tube interne (4) se prolonge longitudinalement par deux branches (4a) diamétralement opposées. Les branches (4a) comprennent une élasticité les autorisant à être écartées ou rapprochées l'une de l'autre, et les extrémités libres des deux branches (4a) forment une pince destinée à pincer la surface externe de la douille (2b) pour se verrouiller sur celle-ci. L'élasticité des branches (4a) permet, en partie, de maintenir le verrouillage du dispositif d'extension (3) sur la douille (2b).

D'une manière avantageuse, les extrémités libres des branches (4a) du tube interne (4) comprennent chacune un ergot (6) agencé sur leur surface interne destinée à être en contact avec la surface externe de la douille (2b). D'une manière correspondante, la surface externe de la douille (2b) de la vis pédiculaire (2) selon l'invention comprend deux logements (6a) diamétralement opposées et aptes à recevoir en engagement les ergots (6) des extrémités libres des branches (4a) du tube interne (4).

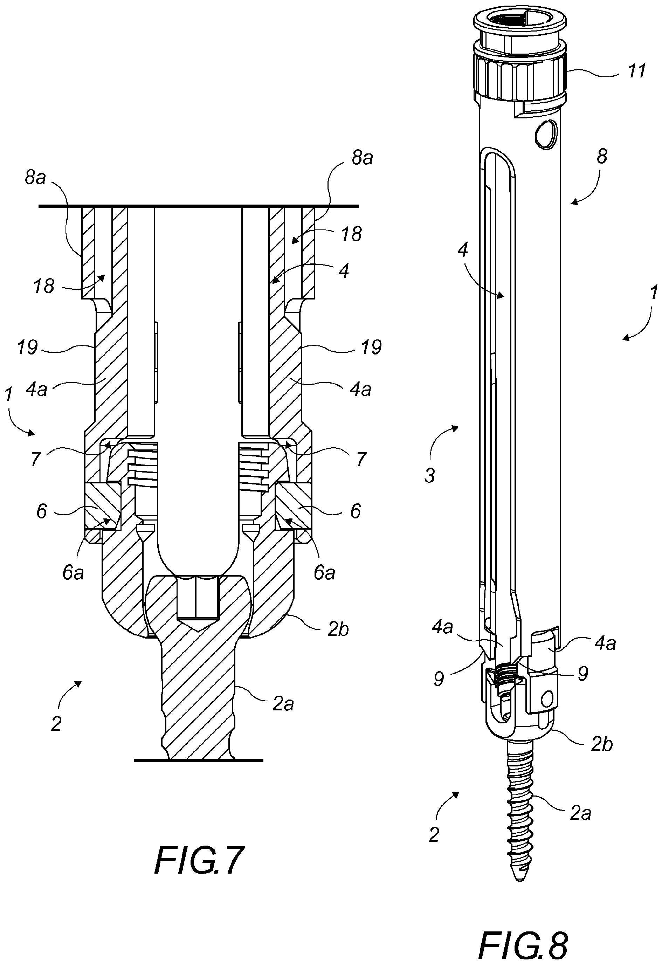

En référence à la figure 7 lorsque les ergots (6) coopèrent avec les logements complémentaires (6a) de la douille (2b), ceux-ci sont aptes à verrouiller le déplacement longitudinal desdites branches (4a) du tube interne (4) par rapport à la surface externe de la douille (2b). En d'autres termes, lorsque les branches (4a) du tube interne (4) sont verrouillées sur la douille (2b), il devient alors nécessaire d'écarter les branches (4a) l'une de l'autre pour sortir les ergots (6) des logements (6a) et pour déverrouiller le dispositif d'extension (3).

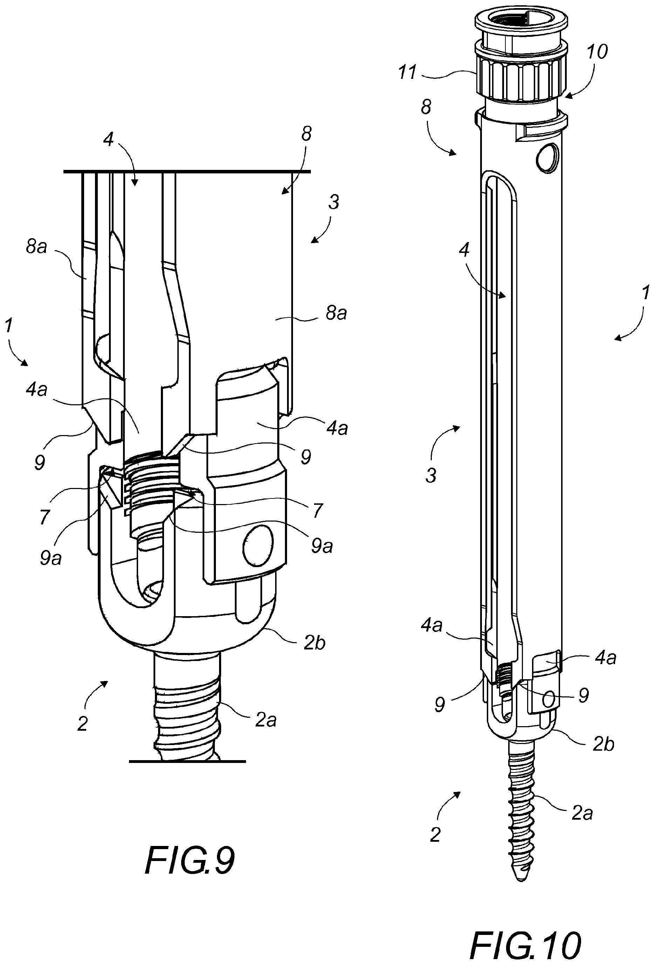

Les branches (4a) du tube interne (4) viennent donc coiffer la douille (2b) (voir figures 8 et 9), et celles-ci présentent, à leurs extrémités libres, un épaulement transversal (7) apte à réaliser une butée contre la douille (2b) d'une vis pédiculaire (2) lorsque ledit dispositif d'extension (3) vient coiffer ladite douille (2b). La coopération entre la douille (2b) et le dispositif d'extension (3) est alors maitrisée.

Le dispositif d'extension (3) comprend ensuite un deuxième élément tubulaire, dit tube externe (8), monté autour du tube interne (4). Le diamètre interne du tube externe (8) est ajusté au diamètre externe dudit tube interne (4) de manière à pouvoir coulisser le long dudit tube interne (4), entre une position dite de non verrouillage, et une position dite de verrouillage d'une vis pédiculaire (2) (voir figures 10, 11, 12).

Le tube externe (8) se prolonge longitudinalement par deux branches (8a) diamétralement opposées et superposées aux branches (4a) du tube interne (4). Les branches (4a) du tube interne (4), ainsi que les branches (8a) du tube externe (8), sont diamétralement opposées et écartées l'une de l'autre de manière à autoriser l'insertion d'une tige de liaison transversale (non représentée) entre les branches (4a, 8a) de chaque tube (4, 8). Cette tige de liaison peut ensuite être glissée d'une manière connue jusqu'à la douille (2b) de la vis pédiculaire (2) lorsque le dispositif d'extension (3) coiffe ladite douille (2b).

Les branches (8a) du tube externe (8) comprennent également une élasticité les autorisant à être écartées ou rapprochées l'une de l'autre de sorte à ne pas gêner l'écartement des branches (4a) du tube interne (4) lorsque le tube externe (8) est dans sa position de non verrouillage.

L'élasticité des branches (8a) du tube externe (8) n'est cependant pas indispensable. En effet, en référence à la figure 7, un jeu (18) subsiste entre le tube interne (4) et le tube externe (8). Ce jeu (18) permet aux branches (4a) du tube interne (4) de s'écarter l'une de l'autre. Un épaulement (19) ménagé sur la surface externe du tube interne (4) permet de supprimer le jeu (18) après verrouillage, c'est-à-dire après que le tube externe (8) ait coulissé autour du tube interne (4) en direction de la vis pédiculaire (2). Avant verrouillage, le tube externe (8) se situe au-dessus dudit épaulement (19), et après verrouillage, il se situe au niveau dudit épaulement (19).

Les extrémités libres des deux branches (8a) du tube externe (8), comme celles du tube interne (4), sont destinées à être en regard de la vis pédiculaire (2) lorsque le dispositif d'extension (3) coiffe la douille (2b) d'une vis pédiculaire (2). Lesdites extrémités libres des branches (8a) du tube externe (8) comprennent chacune des surfaces de contact (9) aptes à venir en appui, lorsque ledit tube externe (8) est coulissé dans sa position de verrouillage, avec des surfaces de contact (9a) complémentaires ménagées sur la douille (2b) de la vis pédiculaire (2) faisant partie de l'ensemble chirurgical (1), et coiffée par ledit dispositif d'extension (3).

Les surfaces de contact (9) de chacune des branches (8a) du tube externe (8) sont inclinées par rapport au plan transversal du tube externe (8), et sont tournées vers l'extérieur de chaque branche (8a), en regard de la direction d'écartement de chaque branche (8a). Les surfaces de contact (9) de l'une des branches (8a) sont diamétralement opposées, symétriques, et en regard des surfaces de contact (9) de l'autre branche (8a).

Les surfaces de contact (9a) de la douille (2b) sont correspondantes aux surfaces de contact (9) des branches (8a) du tube externe (8), et sont inclinées par rapport au plan transversal de ladite douille (2b). Lesdites surfaces de contact (9a) de la douille (2b) sont diamétralement opposées, et sont tournées vers l'intérieur de la douille (2b). Par surfaces de contact (9, 9a), on entend notamment des pans inclinés.

La coopération entre lesdites surfaces de contact (9, 9a) est telle qu'elle va à l'encontre de l'écartement des branches (8a) du tube externe (8). Les branches (8a) du tube externe (8) sont superposées aux branches (4a) du tube interne (4), et le diamètre interne du tube externe (8) est ajusté au diamètre externe du tube interne (4), de sorte que si les branches (8a) du tube externe (8) ne peuvent pas s'écarter l'une de l'autre, celles-ci empêchent également les branches (4a) du tube interne (4) de s'écarter l'une de l'autre. Ainsi, lorsque les branches (4a) du tube interne (4) sont verrouillées sur la surface externe de la douille (2b) de la vis pédiculaire (2), et que le tube externe (8) est coulissé en position de verrouillage, les branches (8a) du tube externe (8) empêchent les branches (4a) du tube interne (4) de s'écarter l'une de l'autre et donc de se déverrouiller. Le dispositif d'extension (3) est alors maintenu d'une manière optimale dans sa position de verrouillage.

Pour maintenir le tube externe (8) dans sa position de verrouillage, et donc pour maintenir le verrouillage du dispositif d'extension (3) sur la douille (2b) de la vis pédiculaire (2), le dispositif d'extension (3) comprend un organe de verrouillage.

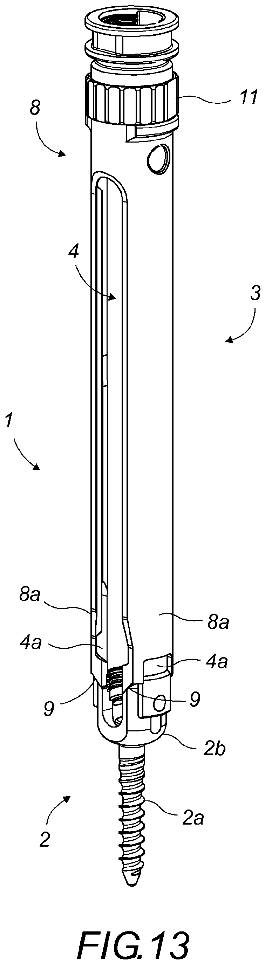

Cet organe de verrouillage peut se présenter sous la forme d'un écrou de serrage (11) apte à coopérer avec une portion externe filetée (10) de l'extrémité du tube interne (4), opposée aux branches (4a). En référence à la figure 13, cette portion externe filetée (10) reçoit l'écrou de serrage (11) qui peut être vissé jusqu'à venir buter contre le tube externe (8) et l'empêcher de coulisser dans sa position de non verrouillage. L'écrou de serrage (11) maintien le tube externe (8) en position de verrouillage.

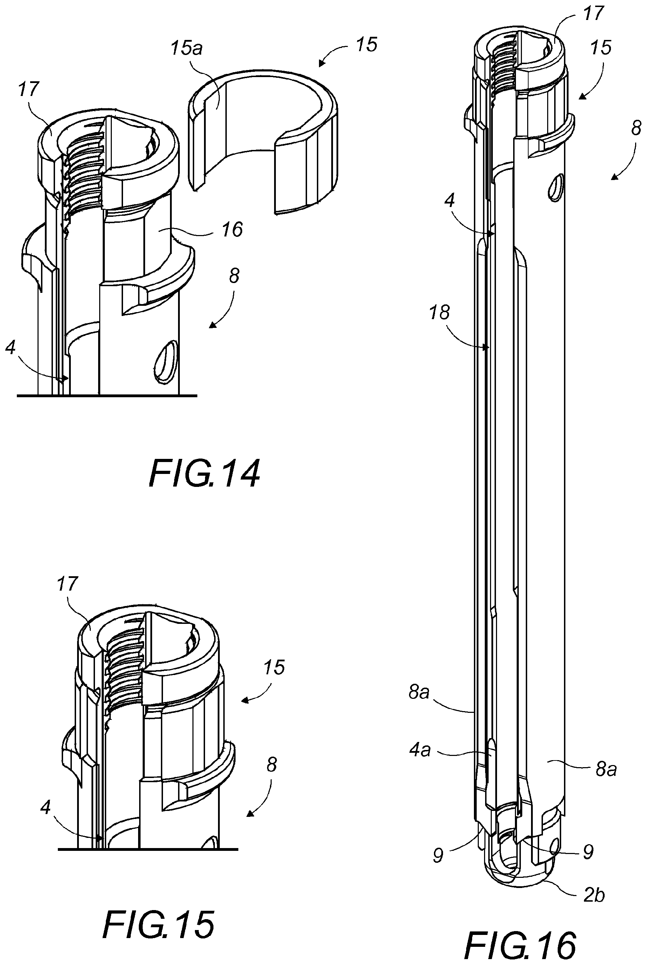

Cet organe de verrouillage peut se présenter par exemple, en référence aux figures 14, 15 et 16, sous la forme d'une bague ouverte (15) pouvant être insérée transversalement à l'extrémité du tube interne (4), opposée aux branches (4a). La bague (15) est insérée entre d'une part, un épaulement (17) ménagé à l'extrémité du tube interne (4) et, d'autre part, l'extrémité du tube externe (8). La bague (15) peut être insérée uniquement lorsque le tube externe (8) est en position de verrouillage. La bague ouverte (15) comprend une élasticité lui permettant de venir s'encliqueter autour du tube interne (4). Son élasticité lui permet de rester en place une fois encliquetée. La bague ouverte (15) présente avantageusement et intérieurement des pans droits (15a) coopérant avec des pans droits (16) complémentaires ménagés sur la surface externe du tube interne (4) de manière à bloquer en rotation ladite bague ouverte (15) une fois encliquetée.

Une fois insérée, la bague (15) empêche le tube externe (8) de revenir en position de non verrouillage. L'ouverture de la bague (15) coïncide avec l'espace disponible entre les deux branches (4a, 8a) des tubes externe (8) et interne (4), et permet ainsi de pouvoir prévoir une ouverture longitudinale (18) tout le long du dispositif d'extension (3). Cette ouverture longitudinale (18) permet de faciliter l'insertion d'une tige de liaison.

Le tube interne (4) et le tube externe (8) comprennent également des moyens de guidage complémentaires aptes à empêcher la rotation dudit tube externe (8) par rapport audit tube interne (4). Ces moyens peuvent être de tout type approprié, tels que par exemple sous la forme d'une nervure longitudinale (non représentée) ménagée sur la surface externe du tube interne (4) et d'une lumière correspondante (non représentée), recevant la nervure, et ménagée dans l'épaisseur du tube externe (8).

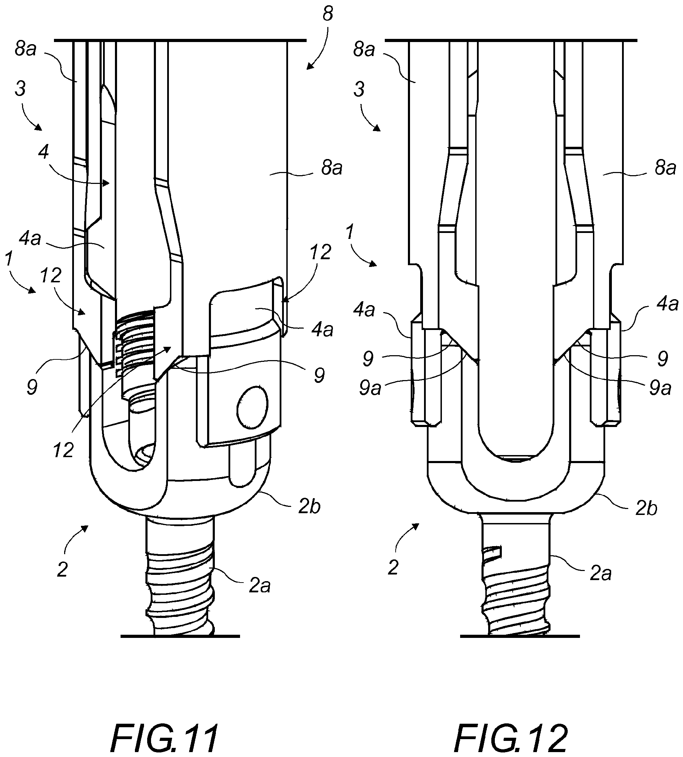

En référence à la figure 11, le guidage peut par exemple être réalisé au niveau des extrémités des branches (8a) du tube externe (8), lesquelles branches (8a) peuvent se diviser en deux doigts (12) agencés de part et d'autre de la branche (4a) du tube interne (4), les deux doigts (12) étant guidés par rapport à cette branche (4a) du tube interne (4). Selon une forme de réalisation particulière, ce sont les deux doigts (12) de chaque branche (8a) du tube externe (8) qui comprennent les surfaces de contact (9) aptes à verrouiller l'écartement desdites branches (8a).

Les branches (4a) du tube interne (4) comprennent une élasticité leur permettant de s'écarter ou de se rapprocher l'une de l'autre. Pour venir coiffer une douille (2b), il faut écarter les branches (4a) du tube interne (4). L'écartement peut être réalisé automatiquement par la coopération de la douille (2b) et des branches (4a), notamment par l'intermédiaire de pente inclinées (13) par exemple (voir figure 4).

Cependant, lorsque les branches (4a) du tube interne (4) comprennent des ergots (6), ou plus particulièrement lorsqu'il est nécessaire d'écarter les branches (4a) du tube interne (4) l'une de l'autre pour déverrouiller le dispositif d'extension (3) de la douille (2b), il peut être nécessaire d'utiliser un outil spécifique (5).

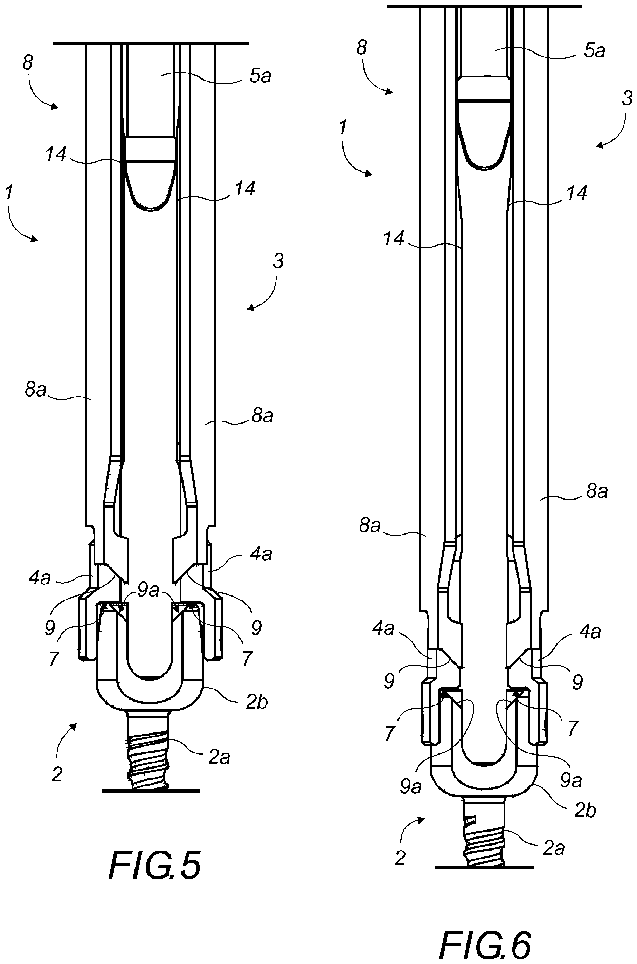

Pour ce faire, les branches (4a) du tube interne (4) comprennent chacune sur leur surface interne un bossage ou un épaulement (14). Lesdits bossages (14) sont diamétralement opposés, et en regard l'un de l'autre de sorte que lorsqu'un outil adapté (5) est inséré à l'intérieur du tube interne (4), celui-ci vient buter contre lesdits bossages (14) pour forcer les branches (4a) du tube interne (4) à s'écarter l'une de l'autre (voir figures 5 et 6).

En référence à la figure 2, l'outil spécifique (5) d'aide à la mise en place du dispositif d'extension (3) peut être de tout type approprié, et peut se présenter sous la forme d'une tige (5a) apte à être introduite dans le tube interne (4) dudit dispositif d'extension (3) pour venir buter contre les bossages ou épaulements (14) et forcer les branches (4a) du tube interne (4) à s'écarter l'une de l'autre.

Ainsi, en référence aux figures 5 et 6, l'outil (5) d'aide à la mise en place du dispositif d'extension (3) peut être utilisé aussi bien pour écarter les branches (4a) du tube interne (4) pour faciliter la mise en place du dispositif d'extension (3) sur la douille (2b), ou bien lors du déverrouillage dudit dispositif d'extension (3).

Comme il ressort de ce qui précède, l'invention fournit un ensemble chirurgical (1) pour le traitement d'instabilités, de fractures ou de déformations d'une colonne vertébrale comprenant un dispositif d'extension (3) et une vis pédiculaire (2) qui permettent de conserver l'accès à la douille (2b) de la vis pédiculaire (2) pendant l'opération chirurgicale, tout en assurant un maintien temporaire optimal dudit dispositif d'extension (3) sur ladite tête de vis (2).

Le dispositif d'extension (3) permet également de faciliter, la mise en place d'une tige de liaison de vis pédiculaires (2), ainsi que la mise en place des organes de verrouillage de ladite tige de liaison dans la douille (2b) de la vis pédiculaire (2), lesquels tige de liaison et organe de verrouillage de la tige étant bien connus de l'homme du métier.

Le dispositif d'extension (3) permet aussi de faciliter la mise en place de tous les instruments nécessaires à l'opération chirurgicale qui permettent la mise en place des implants et d'apporter les corrections à la colonne vertébrale.

## Revendications

1. Ensemble chirurgical (1) comprenant :
- une vis d'ancrage osseux (2) présentant une partie d'ancrage (2a) dans la colonne vertébrale et une tête sous forme d'une douille (2b), et ;
- un dispositif d'extension (3) destiné à venir coiffer la douille (2b) de la vis d'ancrage osseux (2) pour en réaliser un prolongement longitudinal ;
- ledit dispositif d'extension (3) comprend un premier élément tubulaire, dit tube interne (4), de diamètre interne supérieur ou égal au diamètre interne de la douille (2b) de la vis d'ancrage osseux (2), et se prolongeant longitudinalement par deux branches (4a) diamétralement opposées, lesdites branches (4a) comprenant une élasticité les autorisant à être écartées ou rapprochées l'une de l'autre, et les extrémités libres des deux branches (4a) forment une pince apte à pincer la surface externe de la douille (2b) pour se verrouiller sur celle-ci ;
- le dispositif d'extension (3) comprend un deuxième élément tubulaire, dit tube externe (8), monté autour du tube interne (4), et de diamètre interne ajusté au diamètre externe dudit tube interne (4) de manière à pouvoir coulisser le long dudit tube interne (4), entre une position dite de non verrouillage et une position dite de verrouillage de la vis d'ancrage osseux (2), le tube externe (8) se prolongeant longitudinalement par deux branches (8a) diamétralement opposées et superposées aux branches (4a) du tube interne (4), les extrémités libres des deux branches (8a) du tube externe (8) sont en regard des extrémités libres des deux branches (4a) du tube interne (4), ***caractérisé* en ce que** les extrémités libres des deux branches (8a) du tube externe (8) comprennent chacune des moyens de retenue (9) aptes à coopérer, lorsque le dispositif d'extension (3) coiffe la douille (2b) de la vis d'ancrage osseux (2) et lorsque ledit tube externe (8) est coulissé dans sa position de verrouillage, avec des moyens de retenue complémentaires (9a) agencés sur la douille (2b) pour empêcher lesdites branches (8a) du tube externe (8) de s'écarter l'une de l'autre, empêchant ainsi les branches (4a) du tube interne (4) de s'écarter l'une de l'autre de sorte à permettre le maintien du verrouillage du dispositif d'extension (3) sur la douille (2b) de la vis d'ancrage osseux (2).

2. Ensemble chirurgical (1) selon la revendication 1, ***caractérisé* en ce que** les moyens de retenue complémentaires sont des surfaces de contact (9) ménagées aux extrémités des branches (8a) du tube externe (8) et sur la douille (2b), lesdites surfaces de contact (9) étant destinées à venir en appui les unes contre les autres lorsque le tube externe (8) est coulissé dans sa position de verrouillage pour empêcher les branches (8a) du tube externe (8) de s'écarter l'une de l'autre.

3. Ensemble chirurgical (1) selon la revendication 1, ***caractérisé* en ce que** des moyens de fixation complémentaires (6, 6a) aptes à verrouiller le déplacement longitudinal desdites branches (4a) du tube interne (4) par rapport à la surface externe de la douille (2b) sont agencés, d'une part, aux extrémités libres des branches (4a) du tube interne (4), et sur leur surface interne destinée à être en contact avec la surface externe de la douille (2b), et , d'autre part, sur ladite surface externe de la douille (2b).

4. Ensemble chirurgical (1) selon la revendication 1, ***caractérisé* en ce que** dans le dispositif d'extension (3), au moins l'une des branches (4a) du tube interne (4) présente à son extrémité libre un épaulement transversal (7) apte à réaliser une butée contre la douille (2b) de la vis d'ancrage osseux (2) lorsque ledit dispositif d'extension (3) vient coiffer ladite douille (2b).

5. Ensemble chirurgical (1) selon la revendication 1, ***caractérisé* en ce que** le dispositif d'extension (3) comprend un organe de verrouillage apte à maintenir le tube externe (8) en position de verrouillage de la vis d'ancrage osseux (2).

6. Ensemble chirurgical (1) selon la revendication 1, ***caractérisé* en ce que** dans le dispositif d'extension (3), le tube interne (4) et le tube externe (8) comprennent des moyens de guidage complémentaires (12, 4a) aptes à empêcher la rotation du tube externe (8) par rapport au tube interne (4).

7. Ensemble chirurgical (1) selon la revendication 1, ***caractérisé* en ce que** dans le tube d'extension (3), les branches (4a) du tube interne (4) comprennent chacune sur leur surface interne un bossage ou épaulement (14), lesdits bossages (14) étant diamétralement opposés, et en regard l'un de l'autre de sorte que lorsqu'un outil adapté (5) est inséré à l'intérieur du tube interne (4), celui-ci vient buter contre lesdits bossages (14) pour forcer les branches (4a) du tube interne (4) à s'écarter l'une de l'autre.

8. Ensemble chirurgical (1) selon la revendication 7, ***caractérisé* en ce que** le dispositif d'extension (3) comprend un outil (5) sous la forme d'une tige (5a) apte à être introduite dans le tube interne (4) dudit dispositif d'extension (3) pour venir buter contre les bossages ou épaulements (14) et forcer les branches (4a) du tube interne (4) à s'écarter l'une de l'autre.

9. Ensemble chirurgical (1) selon la revendication 1, ***caractérisée* en ce que** dans la vis d'ancrage osseux (2), les moyens de retenue complémentaires (9a) comprennent des pans inclinés formant des surfaces de contact inclinées par rapport au plan transversal de la douille (2b), les surfaces de contact (9a) étant diamétralement opposées et tournées vers l'intérieur de la douille (2b).

## Patentansprüche

1. Chirurgische Anordnung (1), umfassend:
- eine Knochenschraube (2), welche einen Teil zur Verankerung (2a) in der Wirbelsäule und ein Kopfteil in der Form einer Hülse (2b) aufweist, und;
- eine Streckvorrichtung (3), welche zum Schieben über die Hülse (2b) der Knochenschraube (2) vorgesehen ist, um so eine Längsstreckung zu verwirklichen;
- wobei die Streckvorrichtung (3) einen ersten röhrenförmigen Teil, als Innenröhre (4) bezeichnet, mit einem Innendurchmesser von größer oder gleich dem Innendurchmesser der Hülse (2b) der Knochenschraube (2), welcher sich in Längsrichtung auf zwei diametral gegenüberliegende Schenkel (4a) erstreckt, umfasst, wobei diese Schenkel (4a) über eine Elastizität verfügen, die ihnen entweder ein Entfernen voneinander oder ein gegenseitiges Annähern ermöglichen, und die freien Enden der beiden Schenkel (4) bilden eine Zange, welche geeignet ist, die Außenseite der Hülse (2b) einzuklemmen, um auf dieser einzurasten;
- die Streckvorrichtung (3) umfasst einen zweiten röhrenförmigen Teil, als Außenröhre (8) bezeichnet, welche um die Innenröhre (4) herum angebracht ist, mit einem an den Außendurchmesser dieser Innenröhre (4) angepassten Innendurchmesser, um entlang dieser Innenröhre (4) zwischen einer unverriegelten Stellung und einer Verriegelungsstellung der Knochenschraube (2) gleiten zu können, wobei die Außenröhre (8) sich in Längsrichtung auf zwei diametral gegenüberliegende Schenkel (8a) erstreckt, welche die Schenkel (4a) der Innenröhre (4) überlagern; die freien Enden der beiden Schenkel (8a) der Außenröhre (8) stehen den freien Enden der beiden Schenkel (4a) der Innenröhre (4) gegenüber, ***dadurch gekennzeichnet,* dass** die freien Enden der beiden Schenkel (8a) der Außenröhre (8) jeweils Rückhaltemittel umfassen (9), die, wenn die Streckvorrichtung (3) sich über die Hülse (2b) der Knochenschraube (2) schiebt und wenn diese Außenröhre (8) in ihre Verriegelungsstellung gleitet, mit komplementären Rückhaltemitteln (9a) zusammenwirken können, welche auf der Hülse (2b) angeordnet sind, um ein Entfernen dieser Schenkel (8a) der Außenröhre (8) voneinander zu verhindern, wodurch somit ein Entfernen der Schenkel (4a) der Innenröhre (4) voneinander verhindert wird, sodass ein Aufrechterhalten des Einrastens der Streckvorrichtung (3) auf der Hülse (2b) der Knochenschraube (2) ermöglicht wird.

2. Chirurgische Anordnung (1) nach Anspruch 1, ***dadurch gekennzeichnet,* dass** die komplementären Rückhaltemittel Kontaktflächen (9) sind, die an den Enden der Schenkel (8a) der Außenröhre (8) und auf der Hülse (2b) ausgebildet sind, wobei diese Kontaktflächen (9) vorgesehen sind, aneinander anzuliegen, wenn die Außenröhre (8) in ihre Verriegelungsstellung gleitet, um ein Entfernen der Schenkel (8a) der Außenröhre (8) voneinander zu verhindern.

3. Chirurgische Anordnung (1) nach Anspruch 1, ***dadurch gekennzeichnet,* dass** die komplementären Fixierungsmittel (6, 6a), die geeignet sind, eine Längsverschiebung dieser Schenkel (4a) der Innenröhre (4) gegenüber der Außenseite der Hülse (2b) zu blockieren, einerseits an den freien Enden der Schenkel (4a) der Innenröhre (4) sowie auf ihrer Innenseite angeordnet sind, die für den Kontakt mit der Außenseite der Hülse (2b) vorgesehen ist, und andererseits an der Außenseite der Hülse (2b) angeordnet sind.

4. Chirurgische Anordnung (1) nach Anspruch 1, ***dadurch gekennzeichnet,* dass** in der Streckvorrichtung (3) mindestens einer der Schenkel (4a) der Innenröhre (4) an seinem freien Ende einen Quervorsprung (7) aufweist, der geeignet ist, einen Anschlag gegen die Hülse (2b) der Knochenschraube (2) auszuführen, wenn diese Streckvorrichtung (3) auf dieser Hülse (2b) einrastet.

5. Chirurgische Anordnung (1) nach Anspruch 1, ***dadurch gekennzeichnet,* dass** die Streckvorrichtung (3) ein Verriegelungsglied umfasst, das geeignet ist, die Außenröhre (8) in der Verriegelungsstellung der Knochenschraube (2) zu halten.

6. Chirurgische Anordnung (1) gemäß Anspruch 1, ***dadurch gekennzeichnet,* dass** die Streckvorrichtung (3), die Innenröhre (4) und die Außenröhre (8) komplementäre Führungsmittel (12, 4a) umfassen, die geeignet sind, eine Rotation der Außenröhre (8) gegenüber der Innenröhre (4) zu verhindern.

7. Chirurgische Anordnung (1) nach Anspruch 1, ***dadurch gekennzeichnet,* dass** in der Verlängerungsröhre (3) die Schenkel (4a) der Innenröhre (4) jeweils auf ihrer Innenseite eine Erhöhung oder einen Vorsprung (14) umfassen, wobei diese Erhöhungen (14) diametral gegenüberliegend sind und sich so gegenüberstehen, dass, wenn ein passendes Instrument (5) in das Innere der Innenröhre (4) eingeführt wird, es an diesen Erhöhungen (14) anstößt, um ein Entfernen der Schenkel (4a) der Innenröhre (4) voneinander zu erwirken.

8. Chirurgische Anordnung (1) gemäß Anspruch 7, ***dadurch gekennzeichnet,* dass** die Streckvorrichtung (3) ein Instrument (5) in Form eines Stabs (5a) umfasst, der geeignet ist zur Einführung in die Innenröhre (4) dieser Streckvorrichtung (3), um dort an die Erhöhungen oder Vorsprünge (14) anzustoßen und ein Entfernen der Schenkel (4a) der Innenröhre (4) voneinander zu erwirken.

9. Chirurgische Anordnung (1) nach Anspruch 1, ***dadurch gekennzeichnet,* dass** in der Knochenschraube (2) die komplementären Rückhaltemittel (9a) Abschrägungen umfassen, die in Bezug auf die Querebene der Hülse (2b) geneigte Kontaktflächen bilden, wobei die Kontaktflächen (9a) diametral gegenüberliegend und in Richtung des Inneren der Hülse gewandt sind (2b).

## Claims

1. A surgical assembly (1), comprising:
- a bone anchoring screw (2) having an anchoring part (2a) in the spine and a head in the form of a bush (2b), and
- an extension device (3) intended to cap the bush (2b) of the bone anchoring screw (2) to form a longitudinal extension thereof;
- said extension device (3) comprises a first tubular element, called inner tube (4), with an inner diameter greater than or equal to the inner diameter of the bush (2b) of the bone anchoring screw (2), and extending longitudinally by two diametrically opposite branches (4a), said branches (4a) comprising a resiliency allowing them to be separated or brought closer to one another, and the free ends of the two branches (4a) form a clamp able to clamp the outer surface of the bush (2b) to be locked thereon;
- the extension device (3) comprises a second tubular element, called outer tube (8), mounted around the inner tube (4), and with an inner diameter adjusted to the outer diameter of said inner tube (4) so as to be able to slide along said inner tube (4), between a so-called unlocked position and a so-called locked position of the bone anchoring screw (2), the outer tube (8) extending longitudinally by two diametrically opposite branches (8a) and superimposed on the branches (4a) of the inner tube (4), the free ends of the two branches (8a) of the outer tube (8) are facing the free ends of the two branches (4a) of the inner tube (4), ***characterized* in that** the free ends of the two branches (8a) of the outer tube (8) each comprise retaining means (9) able to cooperate, when the extension device (3) caps the bush (2b) of the bone anchoring screw (2) and when said outer tube (8) is slid into its locked position, with complementary retaining means (9a) arranged on the bush (2b) to prevent said branches (8a) of the outer tube (8) from moving away from one another, thus preventing the branches (4a) of the inner tube (4) from moving away from one another so as to allow the maintenance of the locking of the extension device (3) on the bush (2b) of the bone anchoring screw (2).

2. The surgical assembly (1) according to claim 1, ***characterized* in that** the complementary retaining means are contact surfaces (9) arranged at the ends of the branches (8a) of the outer tube (8) and on the bush (2b), said contact surfaces (9) being intended to bear against one another when the outer tube (8) is slid into its locked position to prevent the branches (8a) of the outer tube (8) from moving away from one another.

3. The surgical assembly (1) according to claim 1, ***characterized* in that** the complementary fastening means (6, 6a) able to lock the longitudinal movement of said branches (4a) of the inner tube (4) relative to the outer surface of the bush (2b) are arranged, on the one hand, at the free ends of the branches (4a) of the inner tube (4), and on their inner surface intended to be in contact with the outer surface of the bush (2b), and on the other hand, on said outer surface of the bush (2b).

4. The surgical assembly (1) according to claim 1, ***characterized* in that** in the extension device (3), at least one of the branches (4a) of the inner tube (4) has, at its free end, a transverse shoulder (7) able to produce a stop against the bush (2b) of the bone anchoring screw (2) when said extension device (3) caps said bush (2b).

5. The surgical assembly (1) according to claim 1, ***characterized* in that** the extension device (3) comprises a locking member able to keep the outer tube (8) in the locked position of the bone anchoring screw (2).

6. The surgical assembly (1) according to claim 1, ***characterized* in that** in the extension device (3), the inner tube (4) and the outer tube (8) comprise complementary guiding means (12, 4a) able to prevent the outer tube (8) from rotating relative to the inner tube (4).

7. The surgical assembly (1) according to claim 1, ***characterized* in that** in the extension tube (3), the branches (4a) of the inner tube (4) each comprise, on their inner surface, a boss or shoulder (14), said bosses (14) being diametrically opposite and facing one another such that when an appropriate tool (5) is inserted in the inner tube (4), the latter abuts against said bosses (14) to force the branches (4a) of the inner tube (4) to move away from one another.

8. The surgical assembly (1) according to claim 7, ***characterized* in that** the extension device (3) comprises a tool (5) in the form of a rod (5a) able to be inserted into the inner tube (4) of said extension device (3) to abut against the bosses or shoulders (14) and force the branches (4a) of the inner tube (4) to move away from one another.

9. The surgical assembly (1) according to claim 1, ***characterized* in that** in the bone anchoring screw (2), the complementary retaining means (9a) comprise inclined faces forming contact surfaces inclined relative to the transverse plane of the bush (2b), the contact surfaces (9a) being diametrically opposite and turned toward the inside of the bush (2b).
